# EUROPEAN PATENT SPECIFICATION

(11) **EP 1 672 367 B1**
(45) Date of publication and mention of the grant of the patent: **18.02.2009**
(21) Application number: 04028583.5
(22) Date of filing: 02.12.2004
(51) Int. Cl.: G01N 33/564, G01N 33/68

(54) **Method for screening autoantigen**
Screening-Verfahren für Autoantigen
Méthode de criblage d'autoantigène

(43) Date of publication of application: 21.06.2006
(73) Proprietor: INDUSTRIAL TECHNOLOGY RESEARCH INSTITUTE, Chutung, Hsinchu (TW)
(72) Inventor: Tseng, Tzu-Ling, Daye St., Chiayi City (TW); Cheng, Ping-Fu, Sijhou Township, Changhua County (TW)
(74) Representative: UEXKÜLL & STOLBERG

(56) References cited:
- WO-A-01/79846
- US-A- 5 723 343
- US-A- 5 801 064
- US-A- 5 977 316
- US-A1- 2001 046 713

## Description

### BACKGROUND OF THE INVENTION

### FIELD OF THE INVENTION

The present invention discloses a method for screening autoantigen whereby non-specific antigens are removed using antibodies from normal persons, and then specific autoantigens are obtained using antibodies from patients and identified by using of mass spectrum technology.

### DESCRIPTION OF RELATED ART

People with impaired immune functions are prone to develop immune diseases. The etiology of many human diseases may be traced to our defective immune system in any of the three conditions described below. The first is reduced immunity, lower activity of immune cells, or reduced quantity of immune cells that the body cannot fight off the invading bacteria, virus or mold, and becomes susceptible to contagious diseases, such as common cold, flu, pneumonia, enteritis, or even hepatitis and AIDS. The second condition is immunodeficiency or over-reaction of the immune system where the invading substances are not germs, but tiny pollens or macromolecular proteins in the food ingested, against which the immune system releases large amount of antibodies. Such attack and defense occur in our cells, leading to a chain of reactions which is also called allergy. When real pathogens such as bacteria, virus or mold attack the body at this time, the immune system is no longer able to put up resistance. The third condition of impaired immune system is when the immune cells attack normal cells in the body, called autoimmune disorder as in the case of rheumatoid arthritis, lupus erythematous, and herpes. Such immune diseases arise from our own immune system having identification problem that autoantibodies are produced against body's own cells, resulting in tissue damage and illnesses.

It is now known that autoantibodies are present not just in autoimmune diseases. More and more studies indicate that in the immune response to cancer, autoantigen (from the tumor) and autoantibody (from the body) exist in some cases. Thus the detection of tumor autoantigen that elicits body response may be directed towards and applied in the testing, diagnosis, or prognosis of cancer, and furthermore, in the treatment of disease.

A method commonly used in detecting autoantigen is to react the antibodies in patient's serum with the extract of disease (cancer) related cell lines or pathological tissues using Western blotting. But electrophoresis is limited in the separation and identification of specific proteins due to its poor sensitivity and reproducibility in strongly acidic or basic condition, or if the protein has extremely large or small molecules or very low expression level, or if the protein is membrane protein. A method disclosed in US patent No. 5,723,343 can detect autoantigen through immunoblotting using the sera (containing autoantibodies) from patients with acquired hypoparathyroidism (AH) and extracts of fresh human hypercellular parathyroid glands. But due to the lack of normal controls, low specificity, and limited supply of patient tissue samples, this method does not allow large-scale screening and tends to miss the autoantigen, resulting in low detection of autoantigen. Moreover, this method involves direct retrieval of reacting protein from highly complicated cell extract and sending it into mass spectrometer for analysis. The identification of autoantigen is hence made more difficult due to the complexity of the sample. Thus it is important to develop a systematic and more efficient method for screening autoantigen.

The use of columns containing purified antibodies from individuals suffering from autoimmune diseases for the isolation of autoantigens has been generally described in the art, for example, in WO 01/79846, US 2001/0046713, and US 5,977,316.

### SUMMARY OF THE INVENTION

The present invention discloses a method for screening autoantigen using autoantibodies in patient's body, whereby an autoantigen screening platform is established through the use of chromatographic column packed with autoantibodies, the affinity between autoantibody and autoantigen, and the extract of disease-related cell lines or pathological tissues.

The object of the present invention is to provide an autoantigen screening method comprising the steps of: purifying the antibodies from normal persons and patients respectively; immobilizing the purified antibodies from normal persons and patients in different columns to obtain normal antibody packing column and patient antibody packing column; passing a sample over the normal antibody packing column; passing the sample that has passed through the normal antibody packing column over the patient antibody packing column; displacing the antigens retained in patient antibody packing column; and identifying the antigen obtained from the patient antibody packing column by using a mass spectrometer.

According to the present invention, the method for purifying the antibodies from the serum samples of normal persons and patients respectively comprises, but not limited to, using affinity columns that can capture antibodies, wherein the affinity column comprises, but not limited to, Protein G affinity column or Protein A affinity column, preferably Protein G affinity column.

In one embodiment of the present invention, the sample pretreatment step may be added before passing the sample over the column whereby removing the insoluble matters, such as cell fragments which may cause the column blocked or damage.

The present invention constructs an autoantigen screening method by combining immunoaffinity chromatography, disease-related cell lines, and the maturing mass spectrometry technology, which offers greater screening efficiency and speed than known technologies. Given the continuous supply of cell lines, autoantigens are rarely left out, hence significantly enhancing the chance of detecting autoantigens. The present invention first uses a column packed with antibodies from normal persons to remove non-specific antigens, and then uses a column packed with antibodies from patients to obtain autoantigens having affinity with the antibodies from patients. This method not only enhances the identification of specific autoantigen by autoantibody, which renders subsequent analysis by mass spectrometry more accurate, but it also allows the use of mixture serum sample from a plurality of patients, thereby enriching the incidence of antigen. The resulting autoantigens detected thereof are surely disease related and co-exist in the patient sample, which will work well as a diagnostic tool.

### BRIEF DESCRIPTION OF THE DRAWINGS

FIG. 1 depicts the flow chart for the autoantigen screening method according to the present invention.
FIG. 2 depicts the flow chart for the purification of autoantibody from serum.
FIG. 3 depicts the flow chart for preparation of column containing autoantibodies.
FIG. 4 depicts the flow chart for purification and identification of autoantigens from cell extracts according to the present invention.
FIG. 5 depicts the chromatograms of normal antibody packing column and patient antibody packing column prepared according to the autoantigen screening method of the present invention.

### DETAILED DESCRIPTION OF THE INVENTION

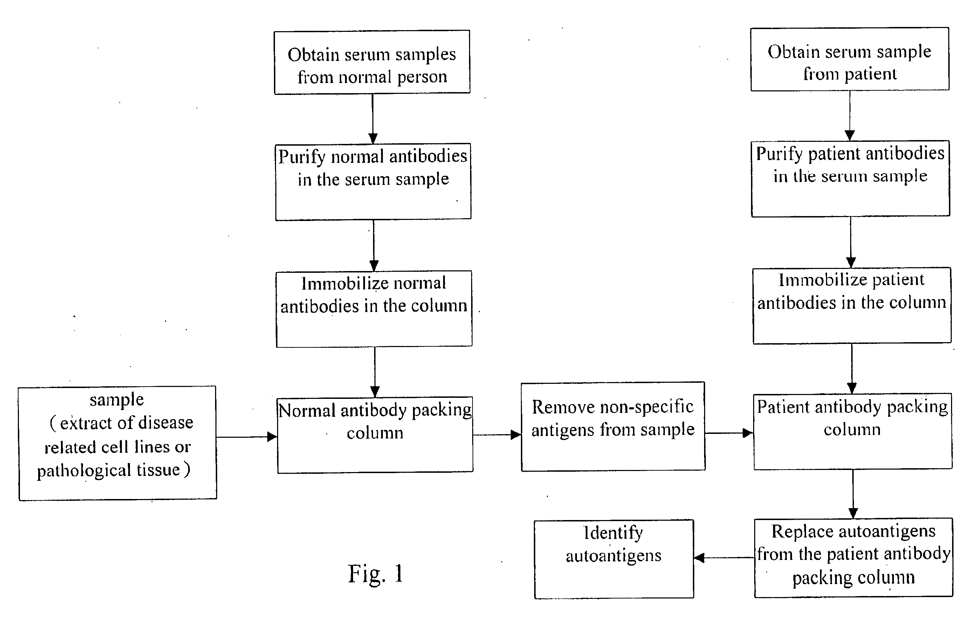

The object of the present invention is to provide a method for screening autoantigen as shown in Fig. 1, comprising the following steps: first obtain serum samples from normal persons and patients and passing the respective samples over affinity column that can capture antibodies (e.g. Protein G affinity column or Protein A affinity column) to purify the antibodies contained in the serum sample; next pack respectively the resulting purified antibodies of the normal person and patient into columns to obtain column containing antibodies from normal persons (normal antibody packing column) and column containing antibodies from patients (patient antibody packing column) in which antibodies are immobilized through the chemical bonding formed between the antibodies and chemical function groups in the column; the aforesaid functional groups include, but not limited to, N-hydroxysuccinimide - (NHS -), cyanobromide - (CNBR-) or epoxy-; obtain a sample which may be the extract of disease related cell lines or pathological tissues; the aforesaid serum sample may be that of a single patient or a mixture sample containing the sera of a plurality of patients. The species of captured antibodies include, but not limited to, immunoglobulin A (IgA), immunoglobulin G (IgG), or immunoglobulin M (IgM).

To continue the procedure, pass the sample from the extract of disease related cell lines or pathological tissues over normal antibody packing column where non-specific antigens are captured and retained in the column through the specific affinity of normal antibodies; this step may be viewed as pre-treatment of sample before patient antibody packing column is used to screen autoantigens in the sample. After non-specific antigens are removed, the sample constitutes only specific antigens. Next, pass the sample over column packed with patient antibodies to screen disease related autoantigen. In light that non-specific antigens have been removed by normal serum antibodies, the autoantigens as screened by patient's autoantibodies are more specific.

Finally, the autoantigens displaced from the patient antibody packing column are subjected to identification by mass spectrometer; the aforesaid identification involves comparing the signals from mass spectrograph with the database to obtain the information on the autoantigens.

The advantages of the present invention are further depicted with the illustration of an example, but the descriptions made in the example should not be construed as a limitation on the actual application of the present invention.

### Example - Screening of liver cancer autoantigen using the method according to the present invention

### Purifying autoantibodies in sera

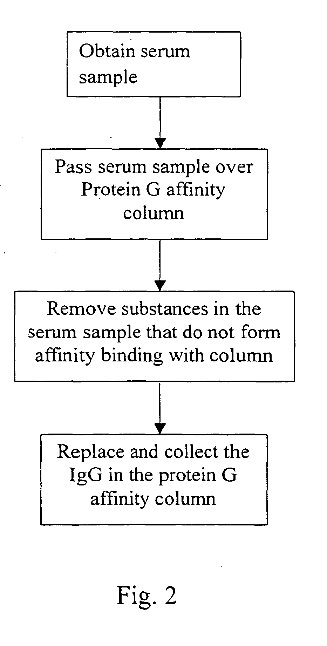

According to the steps of purifying autoantibodies in serum as shown in Fig. 2, dilute the serum with binding buffer (20mM PBS, pH 7.0) at the ratio of 1:10, and then filter the diluted serum using 0.45µm filter membrane to prevent the blockage of column in subsequent steps; next rinse Protein G affinity column with binding buffer ten times the column volume at the rate of 1 ml/min, and then pass the filtered serum sample over the Protein G affinity column at the rate of 0.2 ml/min to retain the antibodies in the column through affinity; rinse the Protein G affinity column again using binding buffer 5-10 times the column volume at the rate of 1 ml/min to remove substances in the serum sample that do not form affinity bonding with the column. Elute antibodies from the column using elution buffer (0.1 M Glycine-HCl, pH 2.7) 2-5 times the column volume at the rate of 1 ml/min and collect the eluted antibodies in a test tube which is added beforehand with 60-200 µl Tris-HCl solution (1M, pH 9.0). Finally displace the sample in coupling buffer (0.2M NaHCO₃, 0.5M NaCl, pH 8.3) to complete the purification of autoantibodies (IgG) in serum sample.

The method according to the present invention requires one normal IgG and patient IgG column each. Thus sera from normal persons and patients should be obtained and subject to the purification step described above.

### Preparation of column containing autoantibodies

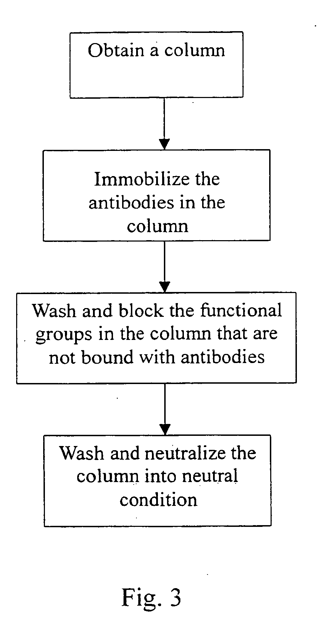

According to the steps of preparing column containing autoantibodies as shown in Fig. 3, pipette one drop of acidification solution (1mM HCl, ice bathed) into a NHS-activated column to prevent the formation of bubbles. After connecting the upper end of column with a syringe or pump, remove the adapter at the bottom of column. Rinse out isopropanol in the column using acidification solution two times the column volume. After repeating the wash step three times, inject the sample containing autoantibodies into the column. Prepare the aforesaid coupling buffer containing purified autoantibodies into a solution with volume equivalent to one time the column volume and concentration of 0.5-10 mg/ml. After passing the aforesaid sample containing autoantibodies over the column, seal the column and let the reaction go on for 15-30 minutes under 25°C or 4 hours under 4°C to immobilize the autoantibodies in the column through chemical bonding.

After the bonding between the autoantibodies and the column, elute the column with blocking buffer (0.5M ethanolamine, 0.5M NaCl, pH 8.3) two time the column volume, and repeat the steps three times. Then rinse the column with washing buffer (0.1M acetate, 0.5M NaCl, pH 4) two times the column volume and also repeat the steps three times. Again elute the column three times using the aforesaid blocking buffer two times the column volume, and then let the column react 15-30 minutes to block and inactivate the functional groups in the column that are not bound with autoantibodies. After completing the blocking reaction, rinse the column three times using the aforesaid washing buffer two times the column volume, followed by eluting the column three times using the aforesaid blocking buffer two times the column volume to make sure all functional groups not bound with autoantibodies are blocked. Again rinse three times the column using washing buffer two times the column volume each time. Finally elute the column with pH neutral buffer 2-5 times the column volume to complete the preparation of column packed with autoantibodies.

### Identification of autoantigens from extract of human hepatoma cells

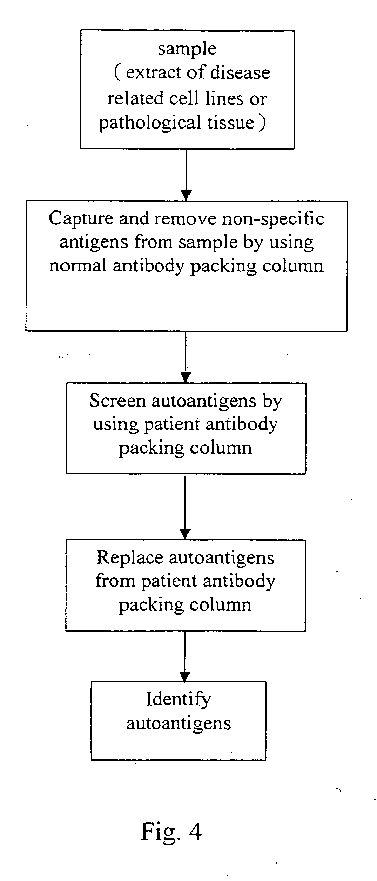

According to the steps shown in Fig. 4, first rinse 2.68 mg of HepG2 C3A cells with culture medium removed with ice-bathed Tris saline solution (50mM Tris pH 7.5, 150 mM NaCl, 1.5 mM PMSF, phosphatase inhibitors) twice, then add in 1ml of Triton^{®} Extraction solution (15mM Tris pH 7.5, 120mM NaCl, 25mM KCl, 2mM EGTA, 0.1mM DTT, 0.5% Triton^{®} X-100, 10µg/ml leupeptin, 0.5mM PMSF, and phosphatase inhibitors) and let it stand for 30 minutes under 4°C. At this time, cells start to decompose and release proteins. Centrifuge (with tabletop centrifuge) the solution at 14,000 rpm under 4°C for 15 minutes to remove solid, insoluble cell structure. Collect the supernatants to carry on immunoaffinity chromatography.

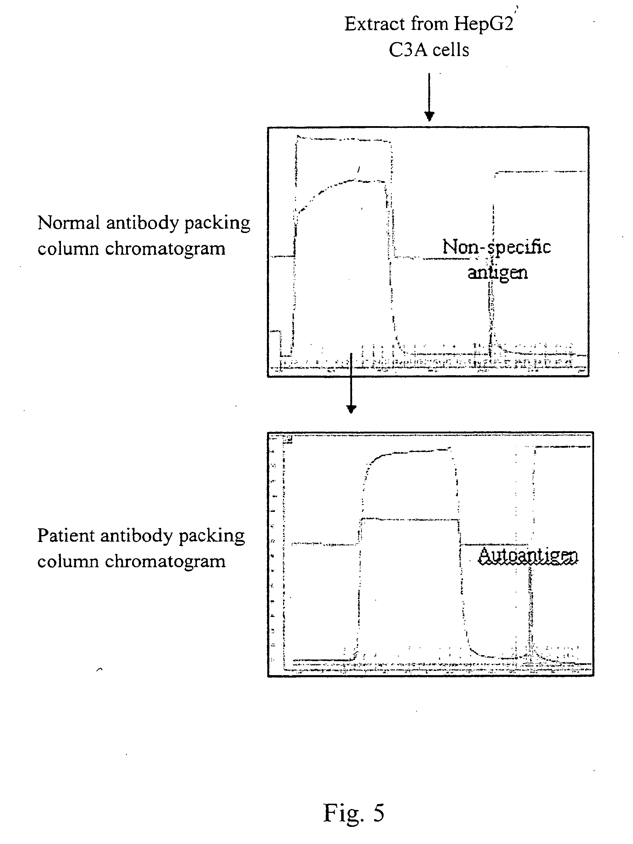

After diluting the cell extract collected with binding buffer at the ratio of 1:10, pass it through 0.45µm filter membrane to prevent the blockage of column in subsequent steps. Prior to injecting the sample into IgG column, rinse the normal and patient antibody packing columns with binding buffer ten times the column volume at the rate of 1 ml/min. Then pass the filtered cell extract over normal antibody packing column at the rate of 0.2 ml/min. Elute the normal antibody packing column with binding buffer 5-10 times the column volume at the rate of 1ml/min. At this time, antigens in the cell extract that are identified and captured by the normal antibodies will be retained in the column. The purpose of this step is to remove non-specific antigens in the HepG2 C3A cells. As a result, the cell extract that has passed through the column is free of non-specific antigens. Inject the resulting cell extract into patient antibody packing column. Elute the column with binding buffer 5-10 times the column volume at the rate of 1 ml/min. At this time, the autoantigens present in the cell extract will be captured by the autoantibodies from patients and retained in the column. As shown in Fig. 5, when cell extract passes over normal antibody packing column, the antigens captured by the normal antibodies are retained in the column, whereas when the cell extract free of antigens that can be identified and captured by normal antibodies, only antigens that can be identified and captured by patient antibodies will be retained by the column. The antigens retained in patient antibody packing column are eluted and collected using elution buffer 2-5 times the column volume at the rate of 1ml/min. Subject the flow-through to protein hydrolysis using trypsin and the resulting peptides are assayed using mass spectrum technology.

To sum up, the present invention uses the autoantibodies in patients to screen autoantigens, whereby a screening platform system is established through the use of chromatographic column packed with autoantibodies, the affinity between autoantibody and autoantigen, and the extract of disease-related cell lines or pathological tissues. This method may be applied in screening specific autoantigen associated with a certain disease. The autoantigen thus screened may be used extensively in disease testing, diagnosis and treatment of immune disorders.

## Claims

1. A method for screening autoantigen, comprising the steps of:
purifying antibodies from normal persons and patients respectively;
immobilizing said purified antibodies of the normal person and patient in different columns to obtain normal antibody packing column and patient antibody packing column;
passing a sample over normal antibody packing column;
passing the sample that has passed through the normal antibody packing column over the patient antibody packing column;
displacing the antigens retained in patient antibody packing column; and
identifying the purified antigens from patient antibody packing column by using a mass spectrometer.

2. The method according to Claim 1, wherein said purified antibodies of normal person and patient are obtained respectively from the serum sample of normal persons and patients by using affinity columns.

3. The method according to Claim 2, wherein said affinity column is Protein-G affinity column or Protein-A affinity column.

4. The method according to Claim 2, wherein said antibodies comprise immunoglobulin A (IgA), immunoglobulin G (IgG) or immunoglobulin M (IgM).

5. The method according to Claim 2, wherein said serum sample of patients is the serum from a single patient.

6. The method according to Claim 2, wherein said serum sample of patients is the mixture serum from a plurality of patients.

7. The method according to Claim 1, wherein the antibodies are immobilized in respective columns via chemical bonding between the antibodies purified from serum and chemical functional groups in the column.

8. The method according to Claim 7, wherein said chemical functional groups comprise N-hydroxysuccinimide- (NHS -), cyanobromide- (CNBr-) or epoxy-.

9. The method according to Claim 1, wherein said sample is extract of disease related cell lines or pathological tissues.

10. The method according to Claim 9, wherein a sample pretreatment step is further added before passing the sample over the normal antibody packing column.

## Patentansprüche

1. Verfahren zum Screening eines Autoantigens, bei dem man:
Antikörper aus normalen Personen bzw. aus Patienten aufreinigt;
die aufgereinigten Antikörper der normalen Person und des Patienten in verschiedenen Säulen immobilisiert, um eine Säule mit einer normalen Antikörper-Packung und eine Säule mit einer Patienten-Antikörper-Packung zu erhalten;
eine Probe über die Säule mit der normalen Antikörper-Packung leitet;
die Probe, die durch die Säule mit der normalen Antikörper-Packung geleitet wurde, über die Säule mit der Patienten-Antikörper-Packung leitet;
die Antigene, die in der Säule mit der Patienten-Antikörper-Packung zurückgehalten wurden, ablöst; und
die gereinigten Antigene aus der Säule mit der Patienten-Antikörper-Packung unter Verwendung einer Massenspektrometrie identifiziert.

2. Verfahren nach Anspruch 1, bei dem die gereinigten Antikörper der normalen Person und des Patienten jeweils aus der Serumprobe von normalen Personen bzw. von Patienten unter Verwendung von Affinitätssäulen erhalten werden.

3. Verfahren nach Anspruch 2, bei dem die Affinitätssäule eine Protein-G-Affinitätssäule oder eine Protein-A-Affinitätssäule ist.

4. Verfahren nach Anspruch 2, bei dem die Antikörper Immunglobulin A (IgA), Immunglobulin G (IgG) oder Immunglobulin M (IgM) umfassen.

5. Verfahren nach Anspruch 2, bei dem die Serumprobe von Patienten das Serum eines einzelnen Patienten ist.

6. Verfahren nach Anspruch 2, wobei die Serumprobe von Patienten eine Mischung aus Serum von einer Vielzahl von Patienten ist.

7. Verfahren nach Anspruch 1, bei dem die Antikörper in den jeweiligen Säulen über chemische Bindung zwischen den aus dem Serum gereinigten Antikörpern und chemischen funktionellen Gruppen in der Säule immobilisiert werden.

8. Verfahren nach Anspruch 7, bei dem die chemischen funktionellen Gruppen N-Hydroxysuccinimid- (NHS-), Cyanobromid-(CNBr-) oder Epoxy- umfassen.

9. Verfahren nach Anspruch 1, bei dem die Probe ein Extrakt aus mit einer Erkrankung in Zusammenhang stehenden Zelllinien oder aus pathologischen Geweben ist.

10. Verfahren nach Anspruch 9, bei dem ferner ein Proben-Vorbehandlungsschritt zugefügt wird, bevor die Probe über die Säule mit der normalen Antikörper-Packung geleitet wird.

## Revendications

1. Procédé de criblage d'autoantigène, comprenant les étapes de :
purification des anticorps provenant respectivement de personnes normales et de patients;
immobilisation desdits anticorps purifiés de la personne normale et du patient dans différentes colonnes pour obtenir une colonne de remplissage d'anticorps normaux et une colonne de remplissage d'anticorps de patient ;
passage d'un échantillon sur une colonne de remplissage d'anticorps normaux ;
passage de l'échantillon qui est passé par la colonne de remplissage d'anticorps normaux sur la colonne de remplissage d'anticorps de patient ;
déplacement des antigènes retenus dans la colonne de remplissage d'anticorps de patient ; et
identification des antigènes purifiés de la colonne de remplissage d'anticorps de patient en utilisant un spectromètre de masse.

2. Procédé selon la revendication 1, dans lequel lesdits anticorps purifiés de personne normale et de patient sont obtenus respectivement à partir de l'échantillon de sérum des personnes normales et des patients en utilisant des colonnes d'affinité.

3. Procédé selon la revendication 2, dans lequel ladite colonne d'affinité est une colonne d'affinité de Protéine-G ou une colonne d'affinité de Protéine-A.

4. Procédé selon la revendication 2, dans lequel lesdits anticorps comprennent l'immunoglobuline A (IgA), l'immunoglobuline G (IgG) ou l'immunoglobuline M (IgM).

5. Procédé selon la revendication 2, dans lequel ledit échantillon de sérum des patients est le sérum provenant d'un seul patient.

6. Procédé selon la revendication 2, dans lequel ledit échantillon de sérum des patients est le mélange de sérum provenant d'une pluralité de patients.

7. Procédé selon la revendication 1, dans lequel les anticorps sont immobilisés dans des colonnes respectives par liaison chimique entre les anticorps purifiés du sérum et des groupes fonctionnels chimiques dans la colonne.

8. Procédé selon la revendication 7, dans lequel lesdits groupes fonctionnels chimiques comprennent les groupes N-hydroxysuccinimide- (NHS -), cyanobromure- (CNBr-) ou époxy-.

9. Procédé selon la revendication 1, dans lequel ledit échantillon est extrait de lignées cellulaires associées à des affections ou de tissus pathologiques.

10. Procédé selon la revendication 9, dans lequel une étape de prétraitement d'échantillon est en outre ajoutée avant de faire passer l'échantillon sur une colonne de remplissage d'anticorps normaux.
